(19) Europäisches Patentamt / European Patent Office / Office européen des brevets

(11) **EP 2 886 043 A1**

(12) **EUROPÄISCHE PATENTANMELDUNG**

(43) Veröffentlichungstag:
24.06.2015   Patentblatt 2015/26

(51) Int Cl.:
*A61B 5/00* (2006.01)    *G06T 7/00* (2006.01)

(21) Anmeldenummer: 13450056.0

(22) Anmeldetag: 23.12.2013

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **a.tron3d GmbH**
**9020 Klagenfurt am Wörthersee (AT)**

(72) Erfinder:
• **Jesenko, Jürgen**
**9161 Maria Rain (AT)**
• **Blassnig, Andreas**
**9020 Klagenfurt (AT)**
• **Angerer, Helmut**
**9020 Klagenfurt (AT)**

(74) Vertreter: **Beer & Partner Patentanwälte KG**
**Lindengasse 8**
**1070 Wien (AT)**

(54) **Verfahren zum Fortsetzen von Aufnahmen zum Erfassen von dreidimensionalen Geometrien von Objekten**

(57)   Verfahren zum Erfassen der dreidimensionalen Oberflächengeometrie von Objekten, insbesondere Zähnen und/oder intraoralen Strukturen, bei dem die folgenden Schritte durchgeführt werden:

1) Laden einer vorhandenen TSDF aus einem Speichermedium;
2) Erfassen eines neuen Tiefenbildes;
3) Laden vorhandener Posen zur geladenen TSDF;
4) Anwenden der geladenen Posen auf das neue Tiefenbild;
e) Überprüfen für jeden durch Anwenden der geladenen Pose transformierten Punkt des Tiefenbildes, ob der Punkt durch die angewandte Pose eine erste definierte Eigenschaft hat und zutreffendenfalls Kennzeichnen des Punktes;
f) Zählen aller gekennzeichneten Punkte im Tiefenbild für jeweils eine Pose;

5) Auswahl vorzugsweise der Pose, welche zu den meisten gekennzeichneten Punkten führt;
6) Erzeugen eines künstlichen Tiefenbildes aus der TSDF mittels der ausgewählten Pose;
7) Anwenden einer ICP-Methode auf das neue Tiefenbild und das künstliche Tiefenbild zum Gewinnen einer weiteren Pose;
8) Überprüfen ob die ICP-Methode aus Schritt 7) zu einer erfolgreichen Registrierung führt;
9) Wenn Schritt 8) erfolgreich ist, Aktualisieren der vorhandenen TSDF mittels des neuen Tiefenbildes und der unter Schritt 7) gewonnenen Pose;10)Wenn Schritt 8) nicht erfolgreich,ist, Wiederholen der Schritte ab Schritt 4).

Fig. 6

EP 2 886 043 A1

**Beschreibung**

Aus dem Stand der Technik sind verschiedene Arten von Intra-oral-Scannern bekannt, mit welchen auf unterschiedliche Arten Tiefenbilder von dreidimensionalen Oberflächengeometrien gewonnen und dann zusammengefügt werden. Wird ein Scanvorgang jedoch unterbrochen, bedarf es verschiedener aufwändiger Verfahren, die häufig nur mit menschlicher Unterstützung möglich sind, um einen unterbrochen Scanvorgang wieder aufzunehmen. Bekannte automatisierte Systeme sind in der Regel erfolglos oder können nur nach vorherigem, manuellem Einschränken des Bereichs, in dem der Scan "neu" angesetzt werden soll, funktionieren.

Der Erfindung liegt daher die Aufgabe zu Grunde, die oben beschrieben Nachteile zu überwinden.

Gelöst wird diese Aufgabe erfindungsgemäß durch ein Verfahren der eingangs genannten Art, bei welcher die folgenden Schritte durchgeführt werden:

> 1) Laden einer vorhandenen TSDF aus einem Speichermedium;
> 2) Erfassen eines neuen Tiefenbildes;
> 3) Laden vorhandener Posen zur geladenen TSDF;
> 4) Anwenden der geladenen Posen auf das neue Tiefenbild;
>
>> a) Überprüfen für jeden durch Anwenden der geladenen Pose transformierten Punkt des Tiefenbildes, ob der Punkt durch die angewandte Pose eine erste definierte Eigenschaft hat und zutreffendenfalls Kennzeichnen des Punktes;
>> b) Zählen aller gekennzeichneten Punkte im Tiefenbild für jeweils eine Pose;
>
> 5) Auswahl vorzugsweise der Pose, welche zu den meisten gekennzeichneten Punkten führt;
> 6) Erzeugen eines künstlichen Tiefenbildes aus der TSDF mittels der ausgewählten Pose;
> 7) Anwenden einer ICP-Methode auf das neue Tiefenbild und das künstliche Tiefenbild zum Gewinnen einer weiteren Pose;
> 8) Überprüfen ob die ICP-Methode aus Schritt 7. zu einer erfolgreichen Registrierung führt;
> 9) Wenn Schritt 8) erfolgreich ist, Aktualisieren der vorhandenen TSDF mittels des neuen Tiefenbildes und der unter Schritt 7. gewonnenen Pose;
> 10) Wenn Schritt 8) nicht erfolgreich ist, Wiederholen der Schritte ab Schritt 4).

Eine TSDF ist dabei die verwendete Form der Notation der dreidimensionalen Daten.

Das neue Tiefenbild kann dabei auf beliebige aus dem Stand der Technik bekannte Art gewonnen werden. Üblicherweise liegen durch ein Tiefenbild 2,5-dimensionale Daten vor.

Posen sind geometrische Transformationen, die gebildet werden, indem Tiefenbilder mittels ICP-Methode ineinander geklappt werden. Durch jedes Tiefenbild, das zu einer TSDF beiträgt oder beigetragen hat, entsteht im Zuge der ICP-Methode üblicherweise eine Pose. Diese Posen werden bevorzugt zusätzlich zu den üblicherweise in einer TSDF hinterlegten Distanzwerten gespeichert und im Zuge des erfindungsgemäßen Verfahrens aufgerufen. Die Reihenfolge der Schritte 1) bis 3) sowie aller gegebenenfalls vorhandenen Unterschritte dieser Schritte ist dabei für die Funktionalität der Erfindung unerheblich. Diese Schritte werden daher bevorzugt gleichzeitig ausgeführt, können aber auch in jeder beliebigen Reihenfolge ausgeführt werden.

Durch das Anwenden der geladenen Posen auf das neue Tiefenbild entstehen Tiefenbilder, in denen die räumlichen Verhältnisse der Punkte der Tiefenbilder zueinander jeweils gleich bleiben, die räumliche Gesamtorientierung des neuen Tiefenbildes aber jeweils anders ist. So kann erfindungsgemäß für jede Pose überprüft werden, welche Punkte des Tiefenbildes eine erste Eigenschaft erfüllen. Diese Eigenschaft ist vorzugsweise, das Punkte des Tiefenbildes, wenn sie in der TSDF notiert werden würden, sich in der Nähe der bereits in der notierten Oberfläche befinden. Man kann diese Eigenschaft beispielsweise "Near Surface" nennen.

Erfindungsgemäß wird dann für jede Pose gezählt wie viele Punkte diese Eigenschaft haben. So kann die Pose gewählt werden, welche dementsprechend den höchsten Zählerstand hat.

Idealerweise ist diese Pose dazu geeignet, das neue Tiefenbild in der TSDF zu notieren. In der Realität stellt das Ermitteln einer Pose mit einer 100%igen Übereinstimmung einen höchst unwahrscheinlichen Zufall dar. Die ermittelte Pose direkt zu verwenden, würde daher die in der TSDF notierte Oberfläche eher verfälschen. Daher wird in einem nächsten Schritt ein künstliches Tiefenbild erzeugt, in dem die ermittelte Pose vereinfacht ausgedrückt "rückwärts" auf die TSDF angewendet wird, um so ein künstliches Tiefenbild mittels der Pose aus der TSDF zu erzeugen. Das reale neue Tiefenbild kann dann in einem weiteren Schritt mit der an sich bekannten ICP-Methode in das künstliche Tiefenbild überführt werden. Dabei entsteht eine weitere Pose.

Da das künstliche Tiefenbild aus der geladenen TSDF stammt, kann mit der dabei ermittelten weiteren Pose das Tiefenbild in die TSDF eingefügt werden. Es wird also versucht, das Tiefenbild in der TSDF zu registrieren.

Um dabei Fehler zu vermeiden und höchstmögliche Genauigkeit zu erreichen wird zusätzlich überprüft, ob es sich dabei um eine gültige bzw. erfolgreiche Registrierung handelt. Eine Registrierung wird dann als gültig betrachtet, wenn die neu in der TSDF hinterlegten Werte mit den bisherigen Werten in der TSDF korrespondieren.

War die Registrierung erfolgreich, wurde der Scan wieder aufgenommen und kann fortgeführt werden.

War die Registrierung nicht erfolgreich, müssen erneut Posen nach den oben beschriebenen Schritten getestet werden.

Gemäß einer bevorzugten Weiterbildung der Erfindung kann daher vor dem Schritt 4) der Schritt 3.1

) Erzeugen künstlicher Posen zusätzlich zu den vorhandenen Posen und Laden der künstlichen Posen

Erfolgen, wobei das Erzeugen der künstlichen Posen durch Anwenden geometrischer Operationen auf die vorhandenen Posen erfolgt.

Dies ist vor allem dann besonders günstig, wenn die vorhandenen Posen sehr verschieden von der aktuellen Ausrichtung des Scanners sind. Dies kann beispielsweise dann vorkommen, wenn der Scann nach der Hälfte des Kieferbogens an den Schneidezähnen abreißt und dann mit einem um 180° gedrehten Scanner versucht wird, den Scan wieder aufzunehmen. Oder wenn der Scan nach einem Abriss auch nur in einer etwas anderen Entfernung wieder aufgenommen werden soll. Daher werden künstliche Posen geschaffen, indem auf die vorhandenen Posen geometrische Operationen angewandt werden. So können die Posen beispielsweise rotiert, zum Objekt geneigt oder auch entfernt oder angenähert werden.

Besonders nach längeren, gegebenenfalls beabsichtigen Unterbrechungen des Scans werden künstliche Posen fast immer hilfreich sein, um zu einer gültigen Registrierung zu kommen. Ohne künstliche Posen kann man vor allem bei kurzen (unbeabsichtigten) Unterbrechungen eine erfolgreiche Wiederaufnahme erzielen, da sich der Scanner dann meistens in einer ähnlichen Position befindet wie vor dem Abriss.

Gemäß eine weiteren bevorzugten Weiterbildung der Erfindung kann nach dem Schritt 1) der Schritt

1.1) Bereitstellen von Bricks der geladenen TSDF

erfolgen.

Dabei wir die TSDF, bzw. ein Raster der TSDF in kleinere "Unter-Raster", die sogenannten Bricks, unterteilt. Diese sind bevorzugt würfelförmig und haben besonders bevorzugt Kantenlängen von $2^n$.

Die Bricks können entweder mit der TSDF geladen werden oder auch durch Unterteilen der TSDF in kleinere Unter-Raster erst bei Bedarf erzeugt werden.

Gemäß einer weiteren bevorzugten Weiterbildung kann nach dem Schritt 1.1) der Schritt

1.2) Bestimmen, welche der bereitgestellten Bricks eine zweite definierte Eigenschaft erfüllen und Markieren dieser Bricks,

erfolgen.

Dafür wird bevorzugt für jeden Brick geprüft, ob sich innerhalb des Bricks Voxel der TSDF befinden, welche die erste definierte Eigenschaft erfüllen. Jeder Brick, der wenigstens ein Voxel mit der ersten Eigenschaft enthält, wird mit der zweiten definierten Eigenschaft markiert.

Wenn dies erfolgt ist, kann das Verfahren gemäß einer besonders bevorzugten Weiterbildung der Erfindung beschleunigt werden, indem, wenn der Schritt 1.2) erfolgt ist, dann statt dem Schritt 4)a) der Schritt

4)a.1) Überprüfen für jeden durch Anwenden der geladenen Pose transformierten Punkt des Tiefenbildes, ob sich der Punkt durch die angewandte Pose in einem Brick mit der zweiten definierten Eigenschaft befindet und zutreffendenfalls Kennzeichnen des Punktes

erfolgt.

So kann je nach gewählter Größe des Bricks das Verfahren um ein Vielfaches schneller durchgeführt werden als mit Schritt 4)a), da die Prüfung sehr viel grober erfolgt.

Der vermeintliche Nachteil, dass eine dann in den Folgeschritten ermittelte Pose gegebenenfalls ungenauer sein könnte, wird wie oben beschrieben, durch die Schritte 6) bis 10) vollständig vermieden, da ohnehin davon ausgegangen wird, dass die ermittelte Pose nicht "perfekt" ist.

Im Folgenden wird ein beispielhafter, mit dem erfindungsgemäßen Verfahren zu betreibender Intra-oral-Scanner umfassender und unter Bezug auf die Zeichnungen näher beschrieben.

Es zeigt:

Fig. 1 ein Schema zum Wechseln von Zuständen von Voxeln einer TSDF,

Fig. 2 eine schematisierte Darstellung eines Sichtkegels einer Kamera im Bezug auf ein Brick,

Fig. 3 eine schematisierte Gegenüberstellung von Normalen in einem Volumensmodell der TSDF und Oberflächennormalen in einem Tiefenbild,

Fig. 4 einen Grafen, der eine Gewichtungsfunktion von Gewichtungswerten der Voxel der TSDF zeigt,

Fig. 5 eine schematisierte Darstellung eines Scannerkopfes mit einem vereinfachten Scannervolumen, welches innerhalb des Scannerkopfes angeordnet ist und

Fig. 6 eine Gegenüberstellung von Punkten eines Tiefenbildes welche mit zwei unterschiedlichen Posen transformiert und mit einer Anzahl von Bricks verglichen werden.

Grundsätzlich kann ein nach dem erfindungsgemäßen Verfahren zu betreibender Intra-oral-Scanner aus zwei wesentlichen Hauptkomponenten aufgebaut sein: Einem Handstück, welches eine intern oder extern hinterlegte Firmware aufweist, und einer Recheneinheit, insbesondere einem Computer, welche über eine entsprechende Software verfügt.

Der Computer kann dabei ein handelsüblicher Computer sein, auf welchem die Software lediglich installiert ist, oder beispielsweise auch ein spezielles Gerät, welches nur dentale Aufgaben erledigen kann. Ersteres hat dabei den Vorteil, dass ein solches System ohne besonderen logistischen Aufwand überall Anwendung finden kann, wo zumindest ein mobiler Computer, wie beispielsweise ein Laptop, zur Verfügung steht. Letzteres hat den Vorteil, dass ein nur für dentale Zwecke gedachter Computer mit besonders geeigneter Hardware versehen werden kann, beispielsweise speziellen Grafikkarten, und so unter Umständen schneller scannen kann.

Die Firmware übernimmt dabei verschiedene Aufgaben. So kann durch sie beispielsweise unter anderem gewährleistet werden, dass bei einem stereometrischen System die Stereobilder zeitsynchron aufgenommen werden. Die Firmware kann außerdem die Sensorparametrierung enthalten, die USB-Datenübertragung steuern (beispielsweise für Videobilder, Parametrierdaten, Status- und Messwerte), Sensoren auslesen (beispielsweise eine Temperatur von Projektor-LEDs, Daten von Lagesensoren, Tastendruckereignisse) und Feedbackelemente ansteuern (beispielsweise mehrfarbige Status-LEDs).

Die Software auf dem Computer beinhaltet beispielsweise die folgenden Softwaremodule:

- ein Kommunikationsmodul, beispielsweise ein USB-Treibermodul, welches als Datenschnittstelle zwischen dem Handstück und dem Computer dient,

- ein Steuerungsmodul, welches im Wesentlichen die physischen Vorgänge beim Scannen ausliest bzw. steuert, insbesondere den bidirektionalen Datenaustausch, d.h. zeitsynchrone Videobildpaare, Belichtungszeit und Verstärkung, Projektor-LED-Temperatur (optional auch weitere Temperaturwerte), Daten von Lagesensoren, verschiedene Zustände der Status-LED (beispielsweise "rot", "gelb" und "grün"), durch Drücken von Tasten des Handstücks ausgelöste Ereignisse (Tastendruckereignisse), Kalibrierdaten der Stereokamera und Parameter für einzelne PC-Softwaremodule;

- ein Vermessungsmodul, welches die erfassten Daten in dreidimensionale Daten umwandelt. Hierfür kann beispielsweise ein $S^3E$-System verwendet werden, das auf einem Kamera/Kamera- und/oder einem Kamera/Projektor-Setup basiert; gegebenenfalls kann dieses Modul auch Mittel zum Verbessern der erfassten Daten enthalten, wobei diese Mittel beispielsweise Daten zü Linsenfehlern, mit welchen die Aufnahmen entzerrt werden, oder Daten zum Dunkelbild, mit welchen der Dunkelstrom kompensiert werden kann, umfassen können.

Ein vereinfacht beschriebener Scanvorgang weist dabei beispieslweise folgende Schritte auf:

- die Projektion eines Lichtmusters auf die Zahnoberfläche und das Zahnfleisch,

- die gegebenenfalls zeitsynchrone Aufnahme von Stereobildpaaren, wobei ein Stereopaar dabei nicht zwingend aus zwei zweidimensionalen Aufnahmen gebildet sein muss. Eine Paarung von Projektionsdaten und einem Bild ist ebenso denkbar,

- Übertragung der erfassten Daten an eine Recheneinheit, beispielsweise über eine USB-Verbindung, optische, drahtlose, elektronische/elektrische und/oder auch mechanische Komponenten,

- Auswerten und Aufbereiten der Daten durch die Recheneinheit.

Wenn vorhanden kann die USB-Verbindung auch die Energieversorgung des Handstückes übernehmen.

Im Folgenden wird kurz das $S^3E$ System erläutert, welches eine bevorzugte Durchführungsform des Vermessungsmoduls ist.

Bei der 3D-Vermessung einer Szene mit dem Vermessungsmodul in Form eines $S^3E$ Systems sind mindestens

zwei Kameras und ein Lichtmusterprojektor mit einem vorab bekannten Lichtmuster erforderlich. Aus diesem Setup kann - unabhängig voneinander sowohl aus einem Kamerabildpaar als auch mit einem Kamerabild und dazu hinterlegtem Lichtmuster - die 3D-Vermessung der Szene durchgeführt werden. Die 3D-Daten werden ineinander übergeführt und liefern so aufgrund der unterschiedlichen Perspektiven insgesamt gesehen jeweils einen dichteren 3D-Datensatz; mit anderen Worten mehr Informationen je realer Flächeneinheit des Objektes. Die beiden Kamerapositionen oder die Position einer der Kameras und des Projektors bezüglich der Szene müssen unterschiedlich sein. Der Abstand zwischen zwei Kameras oder einer Kamera und dem Projektor wird jeweils als Stereobasis bezeichnet. Die Kameras und der Projektor werden geometrisch durch ein Lochkameramodell beschrieben, einzelne Kamerakoordinatensysteme und ein Projektorkoordinatensystem werden in den jeweiligen Brennpunkten (= perspektivisches Projektionszentrum) aufgespannt. Die z-Achsen (= optische Achse) der Kamerakoordinatensysteme werden so ausgerichtet, dass diese normal auf die jeweiligen Bildebenen stehen. Die Position und Orientierung der Koordinatensysteme zueinander muss kalibriert (d.h. bekannt) sein, um über einzelne korrespondierende Bereiche in den jeweiligen Stereobildpaaren oder um über Kamerabild und Projektionsmuster (und die bekannte räumliche Position der Brennpunkte zueinander) die 3D-Koordinaten des jeweiligen Raumpunktes in einem der beiden Kamerakoordinatensysteme triangulieren zu können. Die durch die realen optischen Linsen verursachten Abbildungsfehler (beispielsweise eine Verzerrung der Abbildung, Pupillenfehler oder dergleichen) werden ausgemessen und rechnerisch kompensiert. Die Parametrierung des mathematischen Modells erfolgt im Rahmen des Kalibriervorgangs. Mit einem Kamerabildpaar und einem Bild mit (bekanntem) Projektionsmuster ist eine 3D-Rekonstruktion des jeweiligen Szeneausschnittes möglich, bei welcher im Idealfall für jedes korrespondierende Punktpaar die Koordinaten des jeweiligen Raumpunktes berechnet werden können. Vom zugrundeliegenden Messprinzip her ist beim Stereomessverfahren mit zwei Kameras keine zusätzliche Lichtmusterquelle erforderlich, da zwischen zwei Bildern eines Bildpaares theoretisch auch ohne ein künstliches Muster gleiche Stellen des Objektes erkannt und vermessen werden können. Es wird erfindungsgemäß die Projektion eines (statischen) Zufallslichtmusters eingesetzt, um bei nicht oder nur wenig texturierten Zahnoberflächen möglichst stabil und eindeutig die jeweils korrespondierenden Bildpunkte extrahieren zu können. Weiters kann so auch die 3D-Rekonstruktion durch das Kamera/Projektor-Setup umgesetzt werden. Zusätzlich dient die Projektion auch zur Beleuchtung. Die Korrespondenzfindung (Identifizierung von Punkte-Paaren, also Punkten, die in beiden Bildern eines Stereobildpaares dieselbe Stelle am zu vermessenden Objekt zeigen) in Stereobildpaaren wird vom Prinzip her über die Auswertung der Bildpunktnachbarschaft jedes einzelnen Bildpunktes vorgenommen und erfolgt auf Bruchteile eines Pixels genau (subpixelgenau). Dies kann beispielsweise wie in der WO 2011/041812 A1 beschrieben erfolgen. Sie enthält weiters gekippte Korrelationselemente zum Ausgleich der perspektivischen Verzerrung in den Kamerabildern ("slanted" Korrelation) und wird für mehrere unterschiedliche Auflösungsstufen berechnet. Die resultierende Messgenauigkeit des Systems wird maßgeblich von der Güte der Kalibrierung der Kameras zueinander und der Kalibrierung des Projektors zur Kamera und der Zuverlässigkeit der oben beschriebenen Korrespondenzfindung bestimmt.

Die Ausgangsbasis für die Erstellung eines 3D Modells ist ein erstes Tiefenbild, welches beispielsweise nach wenigstens einer der oben genannten Methoden gewonnen wurde. Dieses erste Tiefenbild definiert einen Ursprung in einem Weltkoordinatensystem. Dabei ist es vollkommen unerheblich, wo genau am zu erfassenden Objekt (oder auch an welcher Stelle des Kieferbogens) mit dem Scan begonnen wird. Im Normalbetrieb liefert dann die Vermessungssoftware das aktuelle Tiefenbild sowie einen Translationsvektor und eine Rotationsmatrix, um dieses Tiefenbild in ein globales Modell einzupassen. Dabei ist das globale Modell die Gesamtheit der bisher erfassten virtuellen Oberflächengeometrie. Die Position einer virtuellen Kamera wandert mit den Posen (das sind die ermittelten geometrischen Transformationn, um ein Tiefenbild in eine TSDF einzufügen, siehe auch weiter unten) des Handstückes mit und liefert die Daten des Gesamtmodells aus der Perspektive des vorherigen Tiefenbilds als virtuelles Bild. Die Berechnung des Translationsvektors und der Rotationsmatrix (also die Registrierung) zum "Einfüllen" eines mit dem Handstück aufgenommenen 3D-Messdatensatz in das globale Modell erfolgt mit Hilfe der Methode ICP (Iterative Closest Point Algorithm, siehe auch: Russinkiewicz und Levoy, "Efficient variants of the iterative closest point algorithm", 2001).

Bei der ICP-Methode wird ausgehend von zwei Punktewolken in mehreren Durchläufen eine Drehung und Verschiebung (geometrische Transfornation) berechnet. Damit ist es möglich, eine der beiden Punktewolken in die Position der jeweils anderen zu bewegen, so dass die Punkte der einen Punktewolke so nahe wie möglich an den Punkten der jeweils anderen Punktewolke liegen. Um zu bestimmen, ob die Punkte einer Punktewolke nahe bei den Punkten einer anderen Punktewolke liegen, wird anhand eines 2D-Tiefenbildes zu jedem Punkt der einen Punktewolke der zugehörige Punkt in der jeweils anderen Punktewolke gesucht, sofern dieser vorhanden bzw. auffindbar ist. So werden Punktepaare gebildet. Der Abstand dieser Punktepaare wird durch die ICP-Methode bei jeder Iteration so weit wie möglich verringert.

Bei einem beispielhaften System mit zwei Kameras und einem Projektor stehen drei mögliche Datenquellen, genannt beispielsweise A, B und C, zur Verfügung. Die Datenquelle A ist dabei ein (stereometrisches) Kamera-Kamera-System mit wenigstens zwei Kameras, die in der aus dem Stand der Technik bekannten stereometrischen Art der Vermessung von Oberflächen Tiefeninformationen liefern. Die Datenquelle B ist ein Kamera-Projektor-System mit einer ersten Kamera (beispielsweise der linken) und einem Projektor, der ein Muster projiziert. Bei diesem System wird die Verzerrung eines aufgenommenen Musters zu einem hinterlegten Muster vermessen. Dies kann analog zu einem stereometrischen System beispielsweise über Disparitäten zwischen dem hinterlegten und dem aufgenommenen bzw. projizierten Muster erfolgen. Die Datenquelle C funktioniert analog zur Datenquelle B mit dem Unterschied, dass eine zweite von der ersten Kamera verschiedene Kamera in Bezug zum Projektor die Tiefeninformationen liefert. Sind für das Handstück mehr als die zwei für ein stereometrisches System notwendigen Kameras vorgesehen, erhöht sich automatisch die Anzahl der verfügbaren Datenquellen. Bei den Datenquellen A und B entspricht das verwendete Kamerakoordinatensystem dem Kamerakoordinatensystem der jeweils ersten Kamera, bei der Datenquelle C ist es das Projektorkoordinatensystem.

Zu jedem Tiefenbild aus den unterschiedlichen Datenquellen wird ein "Confidence"-Bild hinterlegt. Dabei beinhaltet das "Confidence"-Bild eine jedem Bildpunkt eines Tiefenbildes zugeordnete Qualitätsinformation. Diese Qualitätsinformation besagt, wie gering ein errechneter Fehler der Tiefeninformation ist. Die Berechnung der Tiefenbilder geschieht sequentiell aber ausgehend von denselben Kamerabildern, damit der Zeitpunkt der Aufnahme und somit die Position des Handstückes für alle drei Quellen gleich ist. Die Position und Ausrichtung der drei Systeme zueinander ist konstant und wird bei der Kalibrierung berechnet. Die eigentliche Fusionierung der Daten aus den verschiedenen Datenquellen geschieht bei einer Volums-Integration in einer TSDF ("Truncated Signed Distance Function"), einer speziellen Art der Notation von dreidimensionalen Informationen.

Im Stand der Technik verwenden 3D-Scanner entweder eine oder mehrere physische Kameras, um zweidimensionale Daten zu erhalten (üblicherweise zwei oder mehr zweidimensionale Bilder) aus einer oder mehreren verschiedenen Perspektiven und rekonstruieren dreidimensionale Informationen aus den zweidimensionalen Bilden oder auch aus Teilen von diesen. Die rekonstruierte dreidimensionale Information wird dann verwendet, um eine Aufnahme oder einen Teil derselben gegen eine andere Aufnahme oder einen Teil derselben zu registrieren ("frame to frame registration").

Demgegenüber wird gemäß einer bevorzugten Durchführungsform der Erfindung zunächst aus zweidimensionalen Bildern, die mit wenigstens einer physischen Kamera gewonnen wurden, eine erste dreidimensionale Information gewonnen. Dann werden aus dem globalen Modell dreidimensionale Informationen mittels einer virtuellen Kamera errechnet (rekonstruiert bzw. aus den dreidimensionalen Daten "rückprojiziert", wie weiter unten genauer erläutert wird). Dabei werden durch die virtuelle Kamera keine zweidimensionalen Daten geliefert, sondern dreidimensionale Daten, welche direkt aus dem globalen Modell stammen. Diese dreidimensionale Information muss dabei in der räumlichen Ausdehnung (am Objekt) nicht gleich sein wie die dreidimensionale Information, welche aus den zweidimensionalen Bildern der physischen Kamera gewonnen werden. Dann werden die dreidimensionalen Informationen der physischen Kamera gegen die dreidimensionalen Informationen der virtuellen Kamera registriert ("frame to model/global registration"). Da das globale Modell stets die Gesamtheit der bereits erfassten dreidimensionalen Oberflächeninformationen beinhaltet, ist eine derartige Registrierung immer genauer als Registrierungen, wie sie aus dem Stand der Technik bekannt sind.

Nachfolgend wird der oben beschriebene beispielhafte Ablauf noch einmal zusammengefasst:

- Erzeugen wenigstens eines zweidimensionalen Bildes (erste Bildmenge) mittels einer physischen Kamera
- Konstruieren erster dreidimensionaler Informationen aus der ersten Bildmenge
- Erzeugen zweiter dreidimensionaler Informationen mittels der virtuellen Kamera aus dem globalen Modell
- Bestimmen von Korrespondenzen (beispielsweise-mittels der ICP-Methode)
- Bestimmen der geometrischen Transformation (entspricht der Kamerabewegung bzw. Kamerapose)
- Aktualisieren des globalen Modells
- ggf. Erzeugen wenigstens eines neuen zweidimensionalen Bildes mittels der physischen Kamera, vorzugsweise aus einer neuen physischen Position der physischen Kamera.

erfindungsgemäß wird die virtuelle dreidimensionale Oberflächengeometrie des zu erfassenden Objektes in einer TSDF ("truncated signed distance function") hinterlegt. Die TDSF gibt an diskreten Koordinaten (Raster) innerhalb eines definierten Volumens die Distanz entlang einer definierten (Koordinaten-)Achse zur nächsten Oberfläche an. Das Vorzeichen des Funktions- bzw. Distanzwertes gibt Aufschluss darüber, ob sich diese Koordinate des definierten Volumens entlang der Achse vor oder hinter der Oberfläche befindet. Die diskreten Koordinaten des definierten Volumens werden auch Voxel genannt. Bevorzugt sind die Voxel äquidistant. Die tatsächliche Oberfläche ist durch die TSDF implizit gegeben und befindet sich an den Stellen, an denen die Funktion den Wert Null annimmt. Zur exakten Bestimmung der Oberfläche genügt es, lediglich von Voxeln, welche sich nahe der tatsächlichen Oberfläche befinden, Werte zu kennen. Daher wird ab einem definierten Maximalwert ein Voxel als "weit entfernt" markiert. Somit werden die Werte, welche die Funktion annehmen kann, "abgeschnitten" (truncated). Die Größe des Rasters wird bevorzugt groß genug gewählt, um die erfasste Oberfläche beinhalten zu können, vorzugsweise wird dabei auch eine Reserve eingeplant, um beispielsweise eine nicht optimale Positionierung der Oberfläche innerhalb des Rasters zu kompensieren. Die Anzahl der Rasterpunkte insgesamt beeinflusst dabei die Größe der benötigten Rechenressourcen. Grundsätzlich werden bei größeren Rastern die benötigten Ressourcen ebenfalls größer bzw. verlangsamt sich der Scanvorgang bei größerem Raster und gleichbleibenden Ressourcen.

Neben den Distanzwerten können in den Voxeln auch weitere Informationen, beispielsweise zur Qualität des Distanzwertes, hinterlegt werden.

Um neue Informationen aus neu gewonnenen Tiefenbildern in das Modell zu integrieren, muss die Position des Scanners (der physischen Kamera bzw. Kameras) relativ zum Raster bekannt sein. Jedes Voxel wird daraufhin untersucht, ob es von der neuen Messung betroffen ist und dadurch sein Wert mit den neuen Informationen verbessert werden kann.

Wie auch von R. A. Newcombe et al. in "Kinectfusion: Real-time dense surface mapping and tracking" (ISMAR, 2011) beschrieben, arbeitet das TSDF Update rasterorientiert. Daher ist es besonders geeignet, durch einen parallel arbeitenden Prozessor, insbesondere durch einen Grafikprozessor (GPU), durchgeführt zu werden.

Die neuen Tiefendaten werden in das Modell integriert, indem für jeden Rasterpunkt bzw. jedes Voxel unabhängig voneinander folgende Operationen durchgeführt werden können:

1. Ermitteln der Voxel-Position aus Sicht der (virtuellen) Kamera
2. Prüfen, mit welchem Bildpunkt (Pixel) im Tiefenbild ein (bestimmtes) Voxel in der TSDF korrespondiert
3. Integrieren der neuen Distanzinformation, wenn eine Korrespondenz des bestimmten Voxels im Tiefenbild ermittelt wurde und der Tiefenwert gültig ist.

Da der Sichtkegel des Scanners üblicherweise kleiner als das zu erfassende Objekt ist, wird für einen Großteil der Voxel Schritt 3 meist nicht durchgeführt, weil sie sich außerhalb des Sichtkegels des Scanners befinden und es damit keine neuen Informationen für sie gibt.

Wird für ein Voxel eine Distanz zur Oberfläche ermittelt, die größer als der definierte Maximalwert ist, befindet sich das Voxel laut aktueller Messung weit außerhalb im leeren Raum ("Empty Space Update"). Ist der Betrag der Distanz kleiner als der Maximalwert, dann befindet es sich nahe an der gemessen Oberfläche ("Near Surface Update"). Ansonsten ist der Wert weit im negativen Bereich (kleiner als ein Negativmaximum) und das Voxel ist verdeckt und weit hinter der gemessenen Oberfläche und sollte nicht aktualisiert werden. So können je nach Distanz zwei unterschiedliche Arten der Aktualisierung unterschieden werden.

Gemäß einer bevorzugten Durchführungsform des Verfahrens wird die Art der Aktualisierung durch weitere Faktoren beeinflusst. Insbesondere ein Zustand des Voxels vor der Aktualisierung (beispielsweise aus einem vorherigen Durchlauf) wird dabei hinzugezogen. So können beispielsweise Voxel, welche im vorherigen Durchlauf als im leeren Raum befindlich markiert wurden, bis zu einer bestimmten Anzahl von gegenteiligen Aktualisierungen weiterhin als "leer" behandelt werden. Anhand einer Kombination von Kriterien, wie beispielsweise Voxel-Status und neuen Werten, werden für die vorliegende Situation unterschiedliche Strategien gewählt, um die Qualität und Genauigkeit des Distanzwertes zu verbessern oder zumindest nicht mit widersprüchlichen Daten zu verschlechtern.

Generell wird bevorzugt für jedes Voxel die Anzahl der Aktualisierungen gezählt und im Voxel hinterlegt. Diese Anzahl ist ein Gewicht des Voxels. Die Anzahl steigt dabei für Durchläufe, bei denen eine Oberfläche erkannt wurde, bleibt gleich, wenn das Voxel nicht aktualisiert wurde und sinkt, wenn das Voxel bei einem Durchlauf als "leer" ("Empty Space") erkannt wurde. Die Anzahl kann dadurch auch negative Werte erreichen.

Werden mehrere Datenquellen (wie oben beschrieben) verwendet, können deren Tiefenbilder jeweils separat unter Berücksichtigung der verschiedenen Kamerakoordinatensysteme und unterschiedlicher Kameraparameter integriert werden. Eine gleichzeitige Messung mit verschiedenen Systemen wird wie unterschiedliche Messungen aus verschiedenen Blickwinkeln Bild für Bild integriert. Im Idealfall haben dabei die Werte aus allen Systemen die gleiche Qualität. Andernfalls können die Daten verwendet werden, um noch nicht oder nur unzureichend vermessene Bereiche der Oberfläche zu schließen bzw. zu verbessern, während gut erfasste Modellbereiche unverändert bleiben.

Für die TSDF ist es prinzipiell unerheblich, welche und wie viele Quellen pro Iteration ausgewertet werden, jedoch ist es nicht immer zweckmäßig, alle zur Verfügung stehenden Datenquellen (beispielsweise erste Kamera und zweite Kamera, erste Kamera und Projektor, Projektor und zweite Kamera) auszuwerten:

Bei gleichzeitiger Verwendung aller Datenquellen vervielfacht sich der Rechenaufwand für die Aktualisierung der TSDF und damit verringert sich die erreichbare Bildrate erheblich. Die zusätzlichen Daten aus Kamera-Projektor Datenquellen bringen meist nur einen geringen Mehrwert und helfen bevorzugt, für das Stereo-System (erste Kamera und zweite Kamera) schwer zugängliche Bereiche zu erschließen.

Je nach Situation und Systemleistung sind verschiedene, bevorzugte Strategien möglich:

Soll die maximale Bildrate erreicht werden, wird die Auswertung bevorzugt auf jeweils eine Datenquelle pro Iteration begrenzt. Besonders bevorzugt wird dabei das Stereo-System verwendet.

Zusätzlich kann sporadisch (beispielsweise bei jedem 5. Frame) abwechselnd eine der beiden Kamera-Projektor Datenquellen ausgewertet werden.

Zusätzlich oder alternativ kann die Aktivierung weiterer Datenquellen automatisch erfolgen. Steht der Scanner beispielsweise weitgehend still oder bewegt sich nur sehr langsam, kann beispielsweise eine geringere Bildrate toleriert werden.

Weiters kann es der Fall sein, dass bei besonders leistungsstarken Rechnern die Verarbeitung der erfassten Daten schneller erfolgt als das Erfassen selbst, was beispielsweise durch die Bildrate der verwendeten Kameras begrenzt sein kann.

Die Entscheidung, welche Quellen verwendet werden, kann auch anhand von Modell- und Lageinformationen, welche beispielsweise durch Lagesensoren gewonnen werden können, getroffen werden. Bleibt beispielsweise der Inhalt eines Modellbereichs über mehrere Messungen hinweg löchrig, dann kann versucht werden, diesen mit Kamera-Projektor Daten aufzufüllen. Lageinformationen des Scanners bzw. der Kameras und/oder des Projektor, die sich im Scanner befinden, können insbesondere bei der Entscheidung, welche Kamera-Projektor Datenquelle besonders sinnvoll ist, hilfreich sein.

Über den jeweiligen Wert des Gewichts des Voxels (Gewichtungswert) können für jedes einzelne Voxel drei unterschiedliche Voxel-Zustände unterschieden werden:

- Ist der Gewichtungswert Null, wird das Voxel als "unseen" betrachtet.
- Ist der Gewichtungswert größer Null gilt das Voxel als "Near Surface".
- Ist der Gewichtungswert kleiner Null gilt das Voxel als "Empty Space".

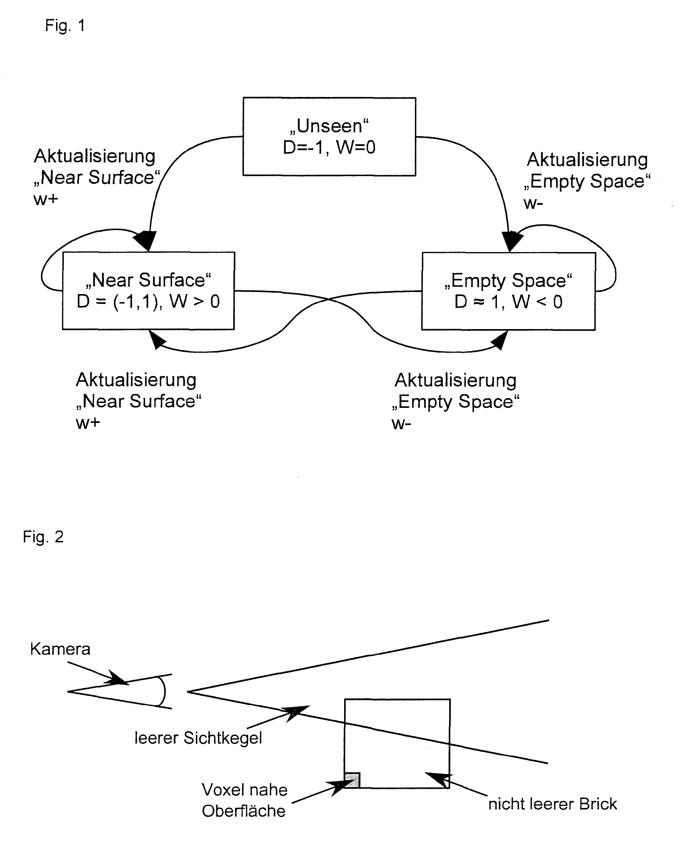

Vergleiche auch Fig. 1, welche skizziert wie die Voxel-Zustände bei "Near Surface" und "Empty Space" Updates behandelt werden und wann sie sich ändern.

Vor der ersten Messung sind alle Werte im Raster unbekannt (Voxel-Zustand: "unseen") und erhalten bei der ersten Aktualisierung sofort einen Voxel-Zustand, einen Distanzwert und Gewichtungswert aus der jeweiligen Aktualisierung.

Befindet sich ein Voxel nahe einer vermuteten Oberfläche (Voxel-Zustand: "Near Surface") und eine neue Aktualisierung bestätigt diese Vermutung, dann wird der neue Distanzwert aus dem ursprünglichen Distanzwert und dem gemessenen Distanzwert ermittelt. Der Gewichtungswert (Voxel-Gewicht) steigt dann.

Eine "Empty Space"-Aktualisierung eines als "Near Surface" markierten Voxels widerspricht folglich dem Gewichtungswert und Zustand dieses Voxels, somit wird der Gewichtungswert verringert. Dies kann eine Zustandsänderung des Voxels zu "Empty Space" bewirken, bei der auch der bis dahin im Voxel hinterlegte Distanzwert mit dem Maximalwert überschrieben wird.

Umgekehrt erhält ein als leer markiertes Voxel (Voxel-Zustand: "Empty Space") einen Gewichtungswert weiter im negativen Bereich.

"Near Surface"-Aktualisierungen lassen den Gewichtungswert ansteigen. Dadurch kann eine Zustandsänderung von "Empty Space" auf "Near Surface" herbeigeführt werden. Dadurch wird der bis dahin im Voxel hinterlegte Maximalwert durch den Distanzwert ersetzt.

Die Genauigkeit der Messwerte in den Tiefenbildern ist nicht vollkommen konstant. Es verringert sich die zu erwartende Genauigkeit, wenn die Oberfläche nicht senkrecht sondern in einem flachen Winkel zum Sehstrahl der Kamera vermessen wird. An Problemstellen sind mehr Messungen nötig und gute Messungen zu bevorzugen, um ein ausreichend genaues Modell erzeugen zu können.

Um auf diese Besonderheit des Scanners einzugehen und die Aussagekraft einer Einzelmessung für das Modell besser bewerten zu können, kann das Voxel-Gewicht gemäß einer bevorzugten Weiterbildung der Erfindung nicht nur als einfacher Zähler verwendet werden.

Je nach Einschätzung der Qualität/Genauigkeit wird der aktuellen Messung ein höheres oder ein geringeres Gewicht zugeordnet. Je geringer dieser Wert ist, desto kleiner ist der Einfluss auf den Distanzwert im Voxel und desto weniger wird der Gewichtungswert des Voxels erhöht. So können beispielsweise Bereiche, die zuvor oft aber nur unter schlechten Bedingungen erfasst wurden, durch nur wenige gute Messungen schnell aufgebessert werden.

Gemäß einer bevorzugten Durchführungsform des Verfahrens kann der Gewichtungswert aus dem Cosinus des Winkels zwischen einer Oberflächennormalen des Tiefenbildes und der Richtung des Sichtstrahls (Einfallswinkel) errechnet werden. Die Oberflächennormale des Tiefenbildes entspricht dabei einer Normalen auf der durch das Tiefenbild erfassten Oberfläche.

Der Gewichtungswert nimmt dementsprechend betragsmäßig, ab je flacher der Sichtstrahl auf die Oberfläche trifft. Die Oberflächennormalen des Tiefenbilds werden bereits bei der ICP-Methode (im Zuge der Registrierung) generiert und stehen für die TSDF Aktualisierung zur Verfügung.

Die intelligente Gewichtung kann erweitert werden, beispielsweise um zusätzliche Qualitätskriterien zu den Tiefenbildern (beispielsweise aus dem "Confidence"-Bild) zu berücksichtigen.

Das beschriebene Verfahren zum Gewichten wird bevorzugt in Verbindung mit dem vorliegenden erfindungsgemäßen Verfahren verwendet, kann aber auch unabhängig davon verwendet werden.

Der von S. F. Frisken and R. N. Perry in "Efficient estimation of 3D euclidean distance fields from 2D range images" (Proceedings of the IEEE Symposium on Volume Visualization änd Graphics) vorgestellte Cliff Ansatz, um Löcher im Modell zu schließen, kann ebenfalls ergänzend angewendet werden. Dazu wird an größeren "Stufen" zwischen Nachbarwerten im Tiefenbild eine Fläche entlang der Sichtstrahlen interpoliert und es werden für das Raster die dazu passenden Distanzwerte erzeugt, um unbekannte Bereiche zu füllen. Diese interpolierten Distanzen werden ihrerseits ohne weitere Berücksichtigung der interpolierten Werte durch gemessene Werte überschrieben.

Gemäß einer bevorzugten Weiterbildung der Erfindung kann zusammen mit der TSDF ein weiteres, kleineres Raster erstellt werden. Die Anzahl der Rasterpunkte an den Kanten des kleineren Rasters ist um einen festgelegten Faktor geringer, und kann beispielsweise einen Wert von 8 oder 16, insbesondere $2^n$, haben. Die kleineren Raster sind jeweils Elemente des Rasters der TSDF und sind bevorzugt Würfel (Bricks), die aus mehreren Voxeln (z.B. 8x8x8, 16x16x16) im TSDF Raster bestehen. Für einen Brick kann angegeben werden, ob dieser Würfel eine Oberfläche beinhalten könnte. Sollte der Brick nur Voxel mit den Voxel-Zuständen "Empty Space" und "unseen" enthalten, dann wird der Brick als leer markiert. Diese Information kann zur Beschleunigung der Oberflächen-Berechnung verwendet werden, um leere Bereiche schnell überspringen zu können ohne jeden einzelnen Voxel prüfen zu müssen.

Um Zeit und Rechenaufwand einzusparen, werden im Zuge der TSDF-Aktualisierung nur alle Bricks im Sichtkegel aktualisiert. Für Bricks außerhalb dieses Bereichs ist keine Änderung zu erwarten, weil sich auch die zugehörigen Voxel der Bricks nicht ändern ("Empty Space Skipping"). Nachdem alle Voxel eines Bricks innerhalb des Sichtkegels aktualisiert wurden, wird untersucht, ob nun eines oder mehrere Voxel nahe einer Oberfläche sind. Wenn das der Fall ist, wird im Brick vermerkt, dass er eine Oberfläche enthält. Dazu werden alle Voxel des Bricks untersucht; auch solche, die sich zwar außerhalb des Sichtkegels befinden, aber zu einem Brick innerhalb des Sichtkegels gehören. Vergleiche hierzu auch Fig. 2.

Die Unterteilung in Bricks bietet die Möglichkeit, auch während des Scan-Betriebs sehr schnell eine grobe Topologie des globalen Modells zu untersuchen. So ist es auch möglich, Flächen, welche nicht Teil des zu erfassenden Objektes sind, zu finden und deren Größe abzuschätzen. Wenn das zu erfassende Objekt beispielsweise ein Kieferbogen ist, kann eine fälschlicherweise erfasste Zunge erkannt werden. Dazu werden alle Bricks, die als eine Oberfläche enthaltend markiert wurden, zu Bündeln zusammengefasst. Alle Bündel aus Oberflächen-Bricks, die nicht direkt mit dem größten Bündel verbunden sind, können als vom Modell isolierte Fragmente angenommen werden.

Die Fragmente können, gemäß einer bevorzugten Weiterbildung der Erfindung, gegebenenfalls nach zusätzlicher Prüfung ihrer Größe, gelöscht werden, indem die Bricks der isolierten Bündel und die Voxel dieser Bricks zurückgesetzt werden. Mit geringem Berechnungsaufwand können so Fragmente, beispielsweise von Zunge, Wange und/oder Verunreinigungen am Abdeckglas, erkannt und entfernt werden.

Wie bereits beschrieben ist bei der TSDF vorgesehen, dass lediglich Distanzwerte mit einem Maximalbetrag vor und hinter der vermuteten Oberfläche bearbeitet werden. Dadurch besteht jedoch die Gefahr, eine andere nahe im Hintergrund liegende Oberfläche (beispielsweise die genüberliegenden Seiten eines Schneidezahns) zu verfälschen. Insbesondere bei Kanten werden dadurch schneewechtenartige Artefakte erzeugt.

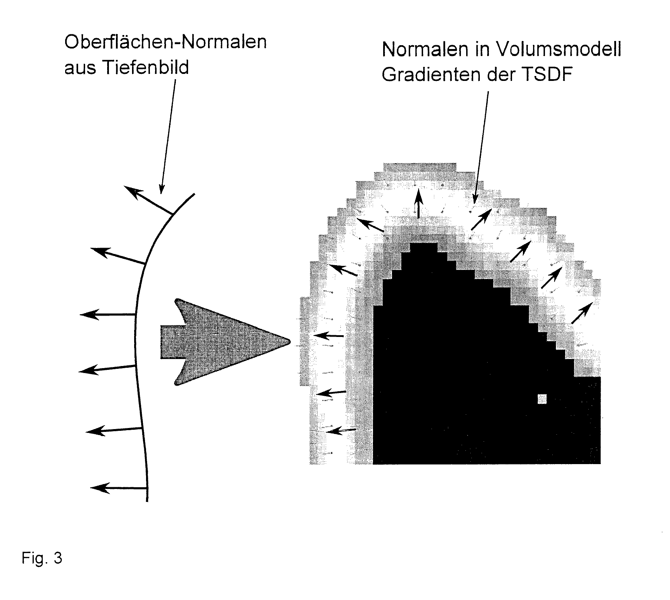

Um diesen Fall zu erkennen, kann die Orientierung der gemessenen Fläche im Tiefenbild (die Oberflächennormale) mit der groben Ausrichtung der in der TSDF hinterlegten Oberfläche an der Stelle des zu bearbeitenden Voxels verglichen werden. Siehe hierzu auch Fig. 3.

Aus dem Vergleich heraus können sich im Wesentlichen drei unterschiedliche Situationen ergeben:

1. Aktualisierung von vorne: Die Oberflächennormalen im Tiefenbild und im Modell sind weitgehend gleich ausgerichtet. Der gemessene Distanzwert aktualisiert den im Voxel vorhandenen Distanzwert.

2. Aktualisierung von hinten: Die Oberflächennormalen im Tiefenbild und im Modell sind weitgehend entgegengesetzt zueinander ausgerichtet. Der neue Distanzwert würde den im Voxel vorhandenen Distanzwert verfälschen. Es kann gegebenenfalls eine weitere (gegenüberliegende) Oberfläche bzw. ein gegenüberliegender Oberflächenabschnitt vermutet werden.

3. Zweideutige Aktualisierung: die Oberflächen stehen schräg zueinander oder sind noch wenig bis gar nicht erfasst worden. In diesem Fall kann beispielsweise der qualitätsabhängige Gewichtungswert des Voxels verringert werden.

Je nach Situation kann nun mit verschiedenen Gewichtungsfunktionen der Einfluss des neuen Messwerts auf den Distanzwert des Voxels bestimmt werden. Für Rückseiten- und zweideutige Fälle wird der Qualitätswert um das resultierende Gewicht verringert, anstatt wie bei Aktualisierungen von vorne erhöht.

Der Gewichtungswert für Aktualisierungen von vorne ist für gute Übereinstimmungen im Wesentlichen 1 und nimmt linear mit dem Cosinus des Einfallswinkel $\alpha$ bis zum Schwellwert $\cos\alpha = -\delta$ bis auf 0 ab. Schlechteren Übereinstimmungen wird weniger "vertraut", daher ist nur ein geringer Einfluss auf den Distanzwert erwünscht.

$$\cos \alpha \in [-1 \dots -\delta] \to \omega_{front} = \frac{(-1)(\delta + \cos \alpha)}{1-\delta} \quad \text{(Formel 1)}$$

Eine Aktualisierung von hinten sollte nicht generell ausgeschlossen werden, da so bestimmte Fragmente nicht aktualisiert werden und daher dauerhaft die erfasste Oberfläche verfälschen können. Stattdessen wird der neue (gemessene) Distanzwert nur zu Voxeln hinzugefügt, die laut ihrem (vorhandenen) Distanzwert weniger als einen Schwellwert $-\delta_{dist}$ hinter der Oberfläche liegen. Je weiter sich das Voxel hinter der Fläche befindet desto weniger wird dem neuen Wert vertraut.

$$\cos \alpha \in [-1 \dots -\delta] \to \omega_{back} = \max(0, \cos \alpha \, (-\delta_{dist} - d_{alt})(1 - |d_{neu}|)) \quad \text{(Formel 2)}$$

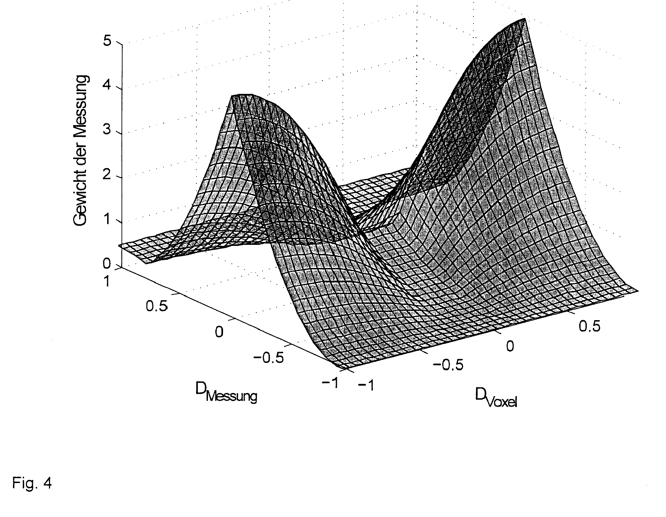

Bei zweideutigen Fällen kann nur anhand der Kombination aus dem Distanzwert im Voxel und dem neuen Distanzwert ein Gewichtungswert ermittelt werden, der schneewechtenartige Verfälschungen dämpft, ohne sich negativ auf andere Stellen der erfassten Oberfläche auszuwirken. Siehe hierzu auch Fig. 4. Die darin dargestellte Funktion w = f($D_{alt}$;$D_{neu}$) wird empirisch so gewählt, dass sie im Nahbereich der Oberfläche symmetrisch ist und ausreichend hohe Gewichtungswerte liefert, um den Distanzwert möglichst nicht zu verfälschen. Unter Nahbereich der Oberfläche wird dabei verstanden, dass beide Distanzwerte (gemessener und vorhandener) nahe Null liegen.

Wird für ein Voxel mit einem geringen, hinterlegten Distanzwert ein sehr hoher neuer Distanzwert erfasst, handelt es sich wahrscheinlich um eine Fehlmessung. Jedoch ist es nicht ratsam, Aktualisierungen in diesem Fall komplett zu verbieten, da dann Fehlmessungen aus früheren Messungen, die mangels Daten noch nicht als solche erkannt werden konnten, und Fehlmessungen durch Fremdkörper, wie beispielsweise eine Zunge, nicht mehr korrigiert werden könnten. Daher ist es sinnvoll, dass auch Voxel mit hohen Gewichtungswerten noch davon beeinflusst werden können.

Das zu erfassende Objekt, beispielsweise ein Kieferbogen, kann während des Scanvorgangs als statisch angenommen werden, wenn während des Scanvorgangs keine Änderungen vorgenommen werden, insbesondere keine Zähne oder Prothesen eingesetzt oder herausgenommen werden. Unerwünschten Objekte, die der Scanner sieht, wie beispielsweise Zunge, Wange, Blut, Speichel und Fremdkörper sind meist beweglich. Diese Annahme kann genutzt werden, um Performance, Qualität und Komfort des Scanvorgang deutlich zu verbessern. Unter der Voraussetzung, dass der Scan auf einem statischen Objekt, beispielsweise einem Zahn, begonnen wird, verschiebt sich die erfasste (virtuelle) Oberfläche nicht innerhalb des globalen Modells bzw. des Rasters der TSDF.

Es ist somit ausgeschlossen, dass (unerwünschte) erfasste Oberflächen in Bereichen, an denen sich der Scanner befindet oder befunden hat, erfasst werden.

Während der Aktualisierung der TSDF können Bereiche (Voxel), die sich innerhalb eines (definierten) Scanner-Volumens befinden, ohne Weiteres dauerhaft als leer ("Empty Space") markiert werden. So können unerwünschte Oberflächen und Artefakte "ausradiert" werden, indem man den Scanner im realen Raum an eine Stelle bewegt, an welcher im korrespondierenden virtuellen Raum eine fehlerhaft erfasste Oberfläche verzeichnet ist. In einer beispielhaften Durchführungsform des Verfahrens kann beispielsweise mit dem Scannerkopf eine zuvor erfasste Zunge weggeschoben werden. Diese Bereiche können danach dauerhaft von der Aktualisierung ausgeschlossen werden. So wird sowohl der Rechenaufwand verringert, als auch ein späteres Auftauchen von neuen Artefakten in diesem Bereich verhindert.

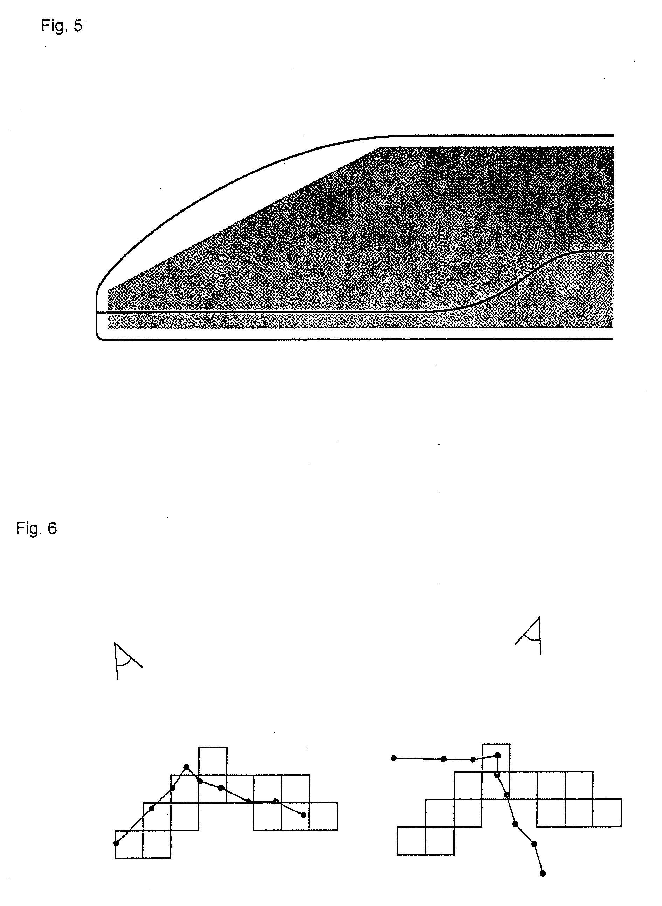

Im Idealfall ist das definierte Scannervolumen (modelliertes Volumen) identisch mit dem Volumen des Scanners, in der Praxis sollte es geringfügig (beispielsweise 5%) kleiner gewählt werden. Durch Vereinfachung der Oberfläche kann es stellenweise ohne weiteres auch deutlich kleiner sein als die tatsächlichen Abmessungen(siehe auch Fig. 5). Wenn das modellierte Volumen exakt gleich groß oder größer ist, könnten gegebenenfalls auch korrekt vermessene Zahnoberflächen gelöscht werden, beispielsweise wenn dort, der Scanner aufliegt.

Gemäß einer bevorzugten Weiterbildung der Erfindung wird Voxeln im radierten Bereich statt einer binären Eigenschaft "radiert"/"nicht radiert" lediglich ein sehr hoher negativer Gewichtungswert zugewiesen, beispielsweise -30000.

In manchen Situationen kann es nützlich sein, die Gewichtungswerte der Voxel zu verringern, um sie schneller aktualisieren zu können. Nicht mehr vorhandene Oberflächen (beispielsweise nach einer Präparation der Zähne) verschwinden im Zuge neuer Aktualisierungen schneller, wenn deren Gewichtungswert gering ist. In "radierten" Bereichen kann sich nur nach Rücksetzen der Gewichtungswerte, d.h. dem Entfernen der Markierung "radiert" bzw. dem Zurücksetzen des eingetragenen Gewichtungswertes, beispielsweise auf "unseen", eine neue Oberfläche bilden.

Gemäß verschiedener bevorzugter Durchführungsform des Verfahrens kann das Rücksetzen beispielsweise auf eine der im Folgenden beschriebenen Arten erfolgen:

- Alle Gewichtungswerte, die höher als ein Gewichtungsschwellwert sind, können auf den Gewichtungsschwellwert gesetzt werden.

- Wenn die Größenverhältnisse der Gewichtungswerte der Voxel untereinander erhalten bleiben sollen, können die Gewichte auch mit einem Faktor skaliert werden (beispielsweise mit dem Faktor 0,5).

- Es kann auch eine beliebige andere Funktion auf die Gewichtungswerte angewandt werden, solange diese Funktion auf alle Gewichtungswerte in der TSDF gleichermaßen angewandt wird.

Verschiedene Kombinationen dieser Varianten sind ebenfalls denkbar.

Das Rücksetzen der Gewichtungswerte kann beispielsweise in einem Pause-Modus geschehen; oder auch bevor eine von einem Speichermedium geladene TSDF aktualisiert wird. Dies kann gegebenenfalls (immer) automatisch beim Laden bzw. Abrufen einer vorhandenen TSDF erfolgen.

Im Folgenden werden einige konkretere Anwendungsbeispiele der Erfindung für das Rücksetzen und/oder Radieren, das bevorzugt in Verbindung mit der vorliegenden Erfindung aber auch unabhängig davon ausgeführt werden kann, noch einmal zusammenfassend kurz erläutert.

- Ein bereits vermessener Kiefer wird nach einer Veränderung, beispielsweise einer (zusätzlichen) Präparation, oder auch dem Einsetzen von prothetischen Elementen, wie beispielsweise einer Überkronung, aktualisiert.

- Ein Teil des Modells wurde fehlerhaft aufgenommen. Es könnten sich z.B. ungewollt Zungen-, Wangen-, Lippen- und/oder Gaumen-Fragmente oder auch Fragmente von zahnärztlichem Gerät, wie beispielsweise von Spiegeln, in der TSDF befinden, welche eine exakte Datenerfassung erheblich erschweren.

- Immer wenn ein Scan abreißt (also vor der Wiederaufnahme) kann der Gewichtungswert automatisch reduziert werden, beispielsweise auf 50%.

- Ein automatisches Rücksetzen bei Scanabriss ist auch dann sinnvoll, wenn der Anwender den Scanner oder den Zahn reinigt (beispielsweise das Entfernen einer Speichelblase) und den entsprechenden Bereich dann nachscannen möchte. Das automatische Rücksetzen hilft dann dabei, dass Fehlmessungen durch Speichel leichter eliminiert werden, ohne dass der Anwender an das Rücksetzen denken muss.

Bei einer bevorzugten Durchführungsform der Erfindung ist es möglich, dass beim Rücksetzen eine Bedienperson einen Hinweis erhält, beispielsweise in Form einer Einfärbung einer für die Bedienperson leicht erfassbaren Visualisierung der TSDF, welcher Sie über das Rücksetzen informiert. Analog können danach auch (neu) aktualisierte Bereiche, beispielweise durch eine Umfärbung, visualisiert werden.

Alternativ kann das Rücksetzen auch erfolgen, ohne dass der Bedienperson ein Hinweis darauf gegeben wird.

Gemäß einer zusätzlichen oder alternativen Durchführungsform der Erfindung können durch die Bedienperson, beispielsweise durch manuelle Interaktion mit einer Maus oder einem Touchscreen, auch nur gewisse Regionen des Modells (innerhalb der visualisierten TSDF) ausgewählt werden. So ist es möglich die Voxel des markierten Bereiches gesondert zu behandeln. Dabei werden die Gewichtungswerte der Voxel im ausgewählten Bereich mit einer der oben genannten Methoden rückgesetzt und die Gewichtungswerte von Voxeln außerhalb des ausgewählten Bereiches bleiben unverändert oder werden um einen anderen Faktor oder auf einen anderen Wert reduziert.

Denkbar wäre auch, dass die Gewichtungswerte der Voxel außerhalb des markierten Bereiches auf einen höheren Wert gesetzt werden, damit sich diese beispielsweise gar nicht mehr verändern können und durch schlechte Messungen, beispielsweise hervorgerufen durch Speichel, somit nicht (mehr) negativ beeinflusst werden können.

In einer weiteren vorteilhaften Durchführungsform der Erfindung ist ein sogenannter Wiederaufnahme-Modus vorgesehen.

Dafür wird zunächst in der ursprünglich erfassten TSDF für jede (erfolgreiche) Aktualisierung die geometrische Transformation (auch Pose genannt) gespeichert.

Nach einem Beenden (oder Pausieren) des Scans wird dann im Wiederaufnahme-Modus ein neues Tiefenbild gewonnen. Dann wird unter Verwendung der gespeicherten Posen versucht, das neue Tiefenbild in der vorhandenen TSDF zu notieren. Die Pose, bei welcher die neuen Distanzwerte in den Voxeln am besten mit den vorhandenen Distanzwerten korrespondieren, wird dann ausgewählt. Für die Auswahl kann beispielsweise die Quadratfehlersumme heran gezogen werden (siehe hierfür auch: "Methode der kleinsten Quadrate", C. F. Gauss).

In einer Weiterbildung der Erfindung können die oben beschriebenen Bricks auch beim Wiederaufnahme-Modus zur Optimierung der Leistung verwendet werden.

Dies kann in einer beispielhaften Durchführungsform der Erfindung in den folgenden Schritten erfolgen:

1. Laden der vorhandenen TSDF aus einem Speichermedium;
2. Bereitstellen der Bricks der vorhandenen TSDF;
3. Zuweisen der Eigenschaft "Near Surface Brick" für alle Bricks in denen wenigstens ein Voxel mit dem Zustand "Near Surface" vorhanden ist;
4. Laden der vorhandenen Posen zur vorhandenen TSDF;
5. Erfassen eines neuen Tiefenbildes;
6. Anwenden der vorhandenen Posen auf das neue Tiefenbild (testweise Transformation des neuen Tiefenbildes mittels der vorhandenen Posen) ;

   a) Überprüfen für jeden transformierten Punkt des Tiefenbildes, ob sich der Punkt durch die angewandte Pose in einem Brick mit der Eigenschaft "Near Surface Brick" befindet und Kennzeichnen des Punktes;
   b) Zählen aller gekennzeichneten Punkte im Tiefenbild für jeweils eine Pose;

7. Auswahl der Pose welche zu den meisten gekennzeichneten Punkten führt;
8. Erzeugen eines künstlichen Tiefenbildes aus der TSDF mittels der ausgewählten Pose;
9. Anwenden der ICP-Methode auf das neue Tiefenbild und das künstliche Tiefenbild;
10. Überprüfen ob die ICP-Methode aus Schritt 9. zu einer erfolgreichen Registrierung führt;
11. Wenn Schritt 10. eine positive Rückmeldung.liefert, Aktualisierung der vorhandenen TSDF mittels des neuen Tiefenbildes und der unter Schritt 10. mit der ICP-Methode gewonnenen Pose;
12. Wenn Schritt 10. eine negative Rückmeldung liefert, wiederholen der Schritte ab Schritt 5.

Der Schritt 3. kann auch unabhängig vom Wiederaufnahme-Modus, beispielsweise direkt beim (ursprünglichen) Erfassen der vorhandenen TSDF erfolgen. In diesem Fall wird die Eigenschaft der Bricks direkt mit den Bricks im Zuge von Schritt 2. geladen und Schritt 3. kann entfallen.

Das unter Schritt 8. erfolgende Erzeugen eines künstlichen Tiefenbildes kann beispielsweise erfolgen, indem aus der TSDF mittels der Methode Ray-Casting aus dem "Blickwinkel" der ausgewählten Pose eine 2,5D-Abbildung der in der TSDF hinterlegten Oberfläche erzeugt wird. Vergleiche hierzu auch: A.Neubauer et al. "Cell-based first-hit ray casting", Proceedings of the Symposium on Data Visualisation 2002, Seiten 77ff.

Die Schritte 6., 6.a) und 6.b) werden in der Fig. 6 noch einmal näher veranschaulicht.

In einer Weiterbildung der Erfindung können neben den Posen, welche mit der TSDF geladen werden (vergleiche auch Schritt 4.), auch sogenannte künstliche Posen unter den Schritten 6. bis 11. verwendet werden. Künstliche Posen werden erzeugt, indem auf vorhandene Posen beliebige Translationen und Rotationen angewendet werden. Dies hat den Vorteil, dass so die Flexibilität im Wiederaufnahme-Modus erhöht wird. So kann beispielsweise ein Scan nach einer 180°-Drehung des Scanners, beispielsweise bei einem Ganzkieferscan nach Absetzen an den Schneidezähnen, einfacher wieder aufgenommen werden, nachdem (wenigstens) eine um 180° gedrehte künstliche Pose erzeugt wurde.

Zusätzlich oder alternativ können beim Erzeugen künstlicher Posen und/oder der Auswahl einer Pose in den Schritten 6. bis 10. weitere Informationen hinzugezogen werden. Insbesondere Lageinformationen aus Beschleunigungs- und Drehratensensoren können Hinweise liefern, welche der aufgezeichneten Posen sich für die Wiederaufnahme eignen können. Diese Informationen können auch genutzt werden, um die besser geeigneten Posen gezielt variieren zu können (Erzeugen künstlicher Posen). Die Zuverlässigkeit dieser Information ist jedoch begrenzt, sie können sogar widersprüchlich werden, wenn sich der Patient bewegt.

Wie bereits weiter oben beschrieben, kann eine Bedienperson durch Hinweise, insbesondere durch visuelle Hinweise, unterstützt werden. Daher ist in einer bevorzugten Weiterbildung der Erfindung vorgesehen, dass die (komplette) Visualisierung der TSDF mit solchen Hinweisen versehen wird.

Wie weiter oben erläutert enthält jedes Voxel der TSDF einen Gewichtungswert. Dieser Gewichtungswert kann für eine Einfärbung einer Visualisierung der TSDF verwendet werden. Da der Gewichtungswert direkt mit der Qualität (bzw. Genauigkeit) der in den Voxeln der TSDF hinterlegten Distanzwerte zusammenhängt, kann eine Bedienperson aus einer mit der Gewichtung verknüpften Einfärbung intuitiv Rückschlüsse auf die Qualität der erfassten Daten ziehen. Daraus ergibt sich eine direkte und leicht verständliche Handlungsanweisung für die Bedienperson, da "schlechte" Bereiche gegebenenfalls erneut gescannt werden müssen.

Diese Hinweise können beispielsweise Farbwerte, unterschiedliche Transparenz, Helligkeit oder dergleichen sein.

Weitere bevorzugte Ausführungsformen der Erfindung sind Gegenstand der übrigen Unteransprüche.

**Patentansprüche**

1. Verfahren zum Erfassen der dreidimensionalen Oberflächengeometrie von Objekten, insbesondere Zähnen und/oder intraoralen Strukturen, bei dem die folgenden Schritte durchgeführt werden:

   1) Laden einer vorhandenen TSDF aus einem Speichermedium;
   2) Erfassen eines neuen Tiefenbildes;
   3) Laden vorhandener Posen zur geladenen TSDF;
   4) Anwenden der geladenen Posen auf das neue Tiefenbild;

   c) Überprüfen für jeden durch Anwenden der geladenen Pose transformierten Punkt des Tiefenbildes, ob der Punkt durch die angewandte Pose eine erste definierte Eigenschaft hat und zutreffendenfalls Kennzeichnen des Punktes;
   d) Zählen aller gekennzeichneten Punkte im Tiefenbild für jeweils eine Pose;

   5) Auswahl vorzugsweise der Pose, welche zu den meisten gekennzeichneten Punkten führt;
   6) Erzeugen eines künstlichen Tiefenbildes aus der TSDF mittels der ausgewählten Pose;
   7) Anwenden einer ICP-Methode auf das neue Tiefenbild und das künstliche Tiefenbild zum Gewinnen einer weiteren Pose;
   8) Überprüfen ob die ICP-Methode aus Schritt 7) zu einer erfolgreichen Registrierung führt;
   9) Wenn Schritt 8) erfolgreich ist, Aktualisieren der vorhandenen TSDF mittels des neuen Tiefenbildes und der unter Schritt 7) gewonnenen Pose;
   10) Wenn Schritt 8) nicht erfolgreich ist, Wiederholen der Schritte ab Schritt 4).

2. Verfahren nach Anspruch 1, **dadurch gekennzeichnet, dass** vor dem Schritt 4) der Schritt

   3.1) Erzeugen künstlicher Posen zusätzlich zu den vorhandenen Posen und Laden der künstlichen Posen

   erfolgt und dass das Erzeugen der künstlichen Posen durch Anwenden geometrischer Operationen auf die vorhandenen Posen erfolgt.

3. Verfahren nach Anspruch 1 oder 2, **dadurch gekennzeichnet, dass** nach dem Schritt 1) der Schritt

   1.1) Bereitstellen von Bricks der geladenen TSDF

   erfolgt.

4. Verfahren nach Anspruche 3, **dadurch gekennzeichnet, dass** nach dem Schritt 1.1) der Schritt

   1.2) Bestimmen, welche der bereitgestellten Bricks eine zweite definierte Eigenschaft erfüllen und Markieren dieser Bricks,

   erfolgt.

5. Verfahren nach Anspruch 4, **dadurch gekennzeichnet, dass** wenn der Schritt 1.2) erfolgt ist, dann statt dem Schritt 4)a) der Schritt

   4)a.1) Überprüfen für jeden durch Anwenden der geladenen Pose transformierten Punkt des Tiefenbildes, ob sich der Punkt durch die angewandte Pose in einem Brick mit der zweiten definierten Eigenschaft befindet und zutreffendenfalls Kennzeichnen des Punktes

   erfolgt.

Fig. 1

„Unseen"
D=-1, W=0

Aktualisierung
„Near Surface"
w+

Aktualisierung
„Empty Space"
w-

„Near Surface"
D = (-1,1), W > 0

„Empty Space"
D ≈ 1, W < 0

Aktualisierung
„Near Surface"
w+

Aktualisierung
„Empty Space"
w-

Fig. 2

Kamera

leerer Sichtkegel

Voxel nahe
Oberfläche

nicht leerer Brick

Fig. 3

Fig. 4

Fig. 5

Fig. 6

Europäisches
Patentamt

European
Patent Office

Office européen
des brevets

**EUROPÄISCHER RECHERCHENBERICHT**

Nummer der Anmeldung

EP 13 45 0056

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2009/074238 A1 (PFISTER HANSPETER [US] ET AL) 19. März 2009 (2009-03-19) * das ganze Dokument * ----- | 1-5 | INV. A61B5/00 G06T7/00 |
| A | US 2007/058829 A1 (UNAL GOZDE [US] ET AL) 15. März 2007 (2007-03-15) * das ganze Dokument * ----- | 1-5 | |

RECHERCHIERTE SACHGEBIETE (IPC)

A61B
G06T

Der vorliegende Recherchenbericht wurde für alle Patentansprüche erstellt

| Recherchenort | Abschlußdatum der Recherche | Prüfer |
|---|---|---|
| Den Haag | 8. April 2014 | Van Dop, Erik |

**ANHANG ZUM EUROPÄISCHEN RECHERCHENBERICHT**
**ÜBER DIE EUROPÄISCHE PATENTANMELDUNG NR.**   EP 13 45 0056

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

08-04-2014

| Im Recherchenbericht angeführtes Patentdokument | Datum der Veröffentlichung | Mitglied(er) der Patentfamilie | Datum der Veröffentlichung |
|---|---|---|---|
| US 2009074238 A1 | 19-03-2009 | KEINE | |
| US 2007058829 A1 | 15-03-2007 | EP 1772826 A2<br>US 2007058829 A1 | 11-04-2007<br>15-03-2007 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2011041812 A1

**In der Beschreibung aufgeführte Nicht-Patentliteratur**

- **A.NEUBAUER et al.** Cell-based first-hit ray casting. *Proceedings of the Symposium on Data Visualisation,* 2002, 77ff